# EUROPEAN PATENT SPECIFICATION

(11) **EP 4 585 146 B1**
(45) Date of publication and mention of the grant of the patent: **14.10.2026**
(21) Application number: 24213150.6
(22) Date of filing: 15.11.2024
(51) Int. Cl.: A61B 5/0538, A61B 5/287, A61B 5/00, A61B 18/14, A61B 5/06, A61B 18/00, A61B 34/20

(54) **IMPEDANCE BASED TRACKING OF CATHETER LOCATION IN RELATION TO A CAVITY WALL**
IMPEDANZBASIERTE VERFOLGUNG DER KATHETERPOSITION IN BEZUG AUF EINE HOHLRAUMWAND
SUIVI BASÉ SUR L'IMPÉDANCE DE L'EMPLACEMENT D'UN CATHÉTER PAR RAPPORT À UNE PAROI DE CAVITÉ

(30) Priority: 09.01.2024 US 202418407808
(43) Date of publication of application: 16.07.2025
(73) Proprietor: Biosense Webster (Israel) Ltd., 2066717 Yokneam (IL)
(72) Inventor: GOVARI, Assaf, 2066717 Yokneam (IL); GLINER, Vadim, 2066717 Yokneam (IL)
(74) Representative: Carpmaels & Ransford LLP

(56) References cited:
- JP-A- 2019 076 709
- US-A1- 2003 130 711

## Description

### TECHNOLOGICAL FIELD

The present disclosure, in some examples thereof, relates to electro-anatomical mapping with an intra-cardiac catheter and more specifically to displaying and/or providing guidance to a physician during mapping.

### BACKGROUND

Intra-body organs include tissue and/or fluids that can vary within different portions of the intra-body organ and that can also vary within different areas of chambers of the intra-body organ, such as different chambers of the heart. Accordingly, tissue proximity, as determined based on electronic signals provided by one or more electrodes may be based on the specific tissue properties at a given location of an intra-body organ, such as at different areas of a heart.

US Patent Number 10,952,637 describes a method which includes receiving, from a probe that comprises electrodes and is positioned inside a cavity in an organ of a patient, (i) proximity signals indicative of proximity of the electrodes to a wall of the cavity, and (ii) position signals indicative of positions of the electrodes within the cavity.

US Patent Application Publication Number 2023/0112251 describes a system includes a catheter and a processor. The catheter includes a distal-end assembly coupled to a distal end of a shaft for insertion into a cavity of an organ of a patient, the distal-end assembly including (i) one or more functional electrodes configured to be placed in contact with wall tissue of the cavity and (ii) a reference electrode configured to be placed in the cavity but not in contact with the wall tissue. The processor is configured to (i) estimate one or more impedances between one or more of the functional electrodes and the reference electrode, and (ii) based on the impedances, determine, for at least a functional electrode from among the one or more functional electrodes, whether the functional electrode is in physical contact with the wall tissue.

US 2003/0130711 A1 provides a method and apparatus for carrying out thermal ablation of target tissue. The apparatus includes an RF ablation device having a multi-electrode electrode assembly designed to be deployed in target tissue, defining a selected volume tissue region lo be ablated, and having infusion channels for infusing a liquid into the target tissue during the ablation process. A control unit in the apparatus is operably connected to an RF energy source, for controlling the RF power level supplied to tl1e electrodes, and to an infusion device, for controlling the rate of infusion of a liquid through the device into the tissue. During both electrode deployment and tissue ablation, impedance. and or temperature measurement made within the tissue are used to control the RF source and infusion device, for optimizing the time and extent of tissue ablation.

### OVERVIEW

Determining and/or indicating a direction toward or away from a tissue wall can assist a medical practitioner in guiding a medical device toward tissue wall around a defining cavity, or away from the tissue wall, or substantially along the tissue wall.

One of the intentions of aspects of the present disclosure is to help a physician guide a catheter and/or a catheter end assembly within a heart chamber without fluoroscopy.

A potential beneficial effect can be helping the physician guide a catheter and/or a distal end assembly of a catheter to an area of interest such as the pulmonary veins.

Tissue impedance is typically larger than that of blood, and larger impedance values are expected to be measured at an electrode which is closer to tissue than at an electrode which is further from tissue.

An example solution includes measuring impedances at one or more electrodes in a body cavity and identifying a gradient in impedance over time for each of the one or more electrodes.

An increase in impedance, e.g., a positive gradient, measured at an electrode indicates that the electrode is moving toward the tissue wall. A decrease in impedance, e.g., a negative gradient measured at an electrode indicates that the electrode is moving away from the tissue wall. For basket type or balloon type catheters, as the physician maneuvers the distal end assembly, some of the electrodes may advance toward the tissue wall, while at the same time other electrodes facing away from the direction of movement, will move away from tissue wall. It may be useful for the physician to receive indication as to which electrodes are approaching the tissue walls. In addition, it may also be useful of the physician to receive indication as to which electrodes are moving in a direction away from tissue. Providing this information in real-time may help the physician maneuver in the chamber and orient the distal end assembly in a desired orientation, for example to reach a region of interest.

In some cases, for example entrance into an ostium may be identified when electrodes positioned circumferentially indicate a sharp increase in impedance while electrodes at the distal end of the distal end assembly indicate a relatively lower increase in impedance or no increase in impedance.

In some examples, when the electrodes are distributed in three dimensions (3D) within a body cavity, it is possible to calculate the 3D gradient of increasing impedance over time using corresponding to a direction toward a surface of the tissue in 3D using data collected from more than one electrode.

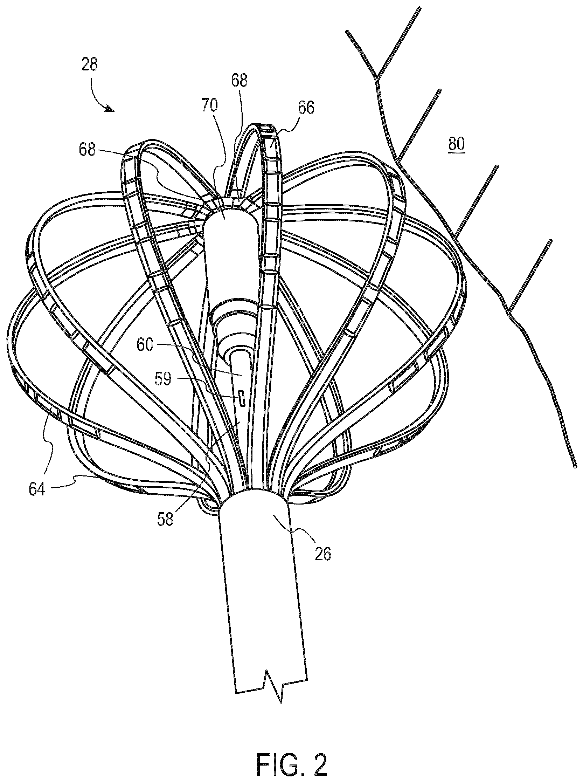

In some examples, a medical device, such as the basket-shaped distal end assembly 28 of Figure 2 is displayed to a physician. While the physician is maneuvering the catheter within the heart chamber, a display shows a virtual representation of the distal end assembly at its current orientation and location. This virtual representation can be shown together with a map of the chamber that has been modeled.

In some examples the display may optionally display increasing impedance over time by coloring or otherwise marking (e.g. brightness) the display of an electrode corresponding to its impedance gradient over time. A physician can see which electrodes are approaching the tissue wall based on their color/brightness/other marking and maneuver the distal end accordingly.

In such examples, the display may optionally also display a direction at which the gradient is the largest, e.g. the direction at which the distal end assembly is approaching the tissue wall at the fastest rate, for example as an arrow pointing toward tissue surface.

In some examples, the direction or arrow may be displayed in a direction away from the tissue surface, or perpendicular to the direction to the tissue surface, or approximately parallel to the tissue surface.

It is also noted that the impedance gradient may be calculated and/or displayed by also taking into account measurements of locations in space of receiving electrodes attached to the medical device, such as distal end assembly 28 of Figure 2, using one or more position sensors.

It is also noted that the impedance gradient may be calculated and/or displayed simultaneously with measuring a location of the medical device, such as the basket-shaped electrode assembly 28 of Figure 2, using one or more position sensors, such as magnetic based Single Axis Sensors (SASs).

### BRIEF DESCRIPTION OF THE DRAWING(S)

Some examples of the disclosure are herein described, by way of example only, with reference to the accompanying drawings. With specific reference now to the drawings in detail, it is stressed that the particulars shown are by way of example and for purposes of illustrative discussion of examples of the disclosure. In this regard, the description taken with the drawings makes apparent to those skilled in the art how examples of the disclosure may be practiced.

In the drawings:
Figure 1 is a simplified illustration of a catheter-based electrophysiology mapping and ablation system, according to examples of the presently disclosed subject matter;
Figure 2 is a more detailed isometric view of an expandable end assembly on the distal tip of the catheter of Figure 1;
Figure 3 is a simplified qualitative illustration showing impedance measured by an electrode in a body cavity as a function of distance from the cavity wall tissue according to an aspect of the disclosure;
Figure 4 is a simplified flow chart illustration of a method of indicating movement of a receiving electrode of a distal end assembly with respect to a tissue cavity wall according to an aspect of the disclosure; and
Figures 5A and 5B are views of the end assembly of the catheter of Figure 2 as displayed on a display, and displaying a direction based on a spatial gradient superposed on the display of the expandable catheter according to an aspect of the disclosure.

### DETAILED DESCRIPTION OF EXAMPLES

The present disclosure, in some examples thereof, relates to electro-anatomical mapping with an intra-cardiac catheter and more specifically to displaying and/or providing guidance to a physician during mapping.

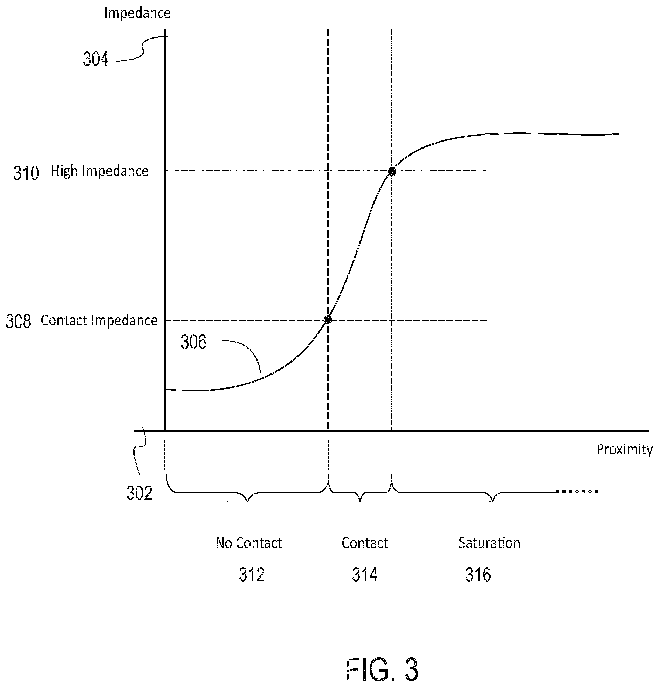

For purposes of better understanding some examples of the present disclosure, as illustrated in Figures 3, 4 and 5A-5B of the drawings, reference is first made to the construction and operation of a catheter-based position-tracking system which includes an expandable end assembly.

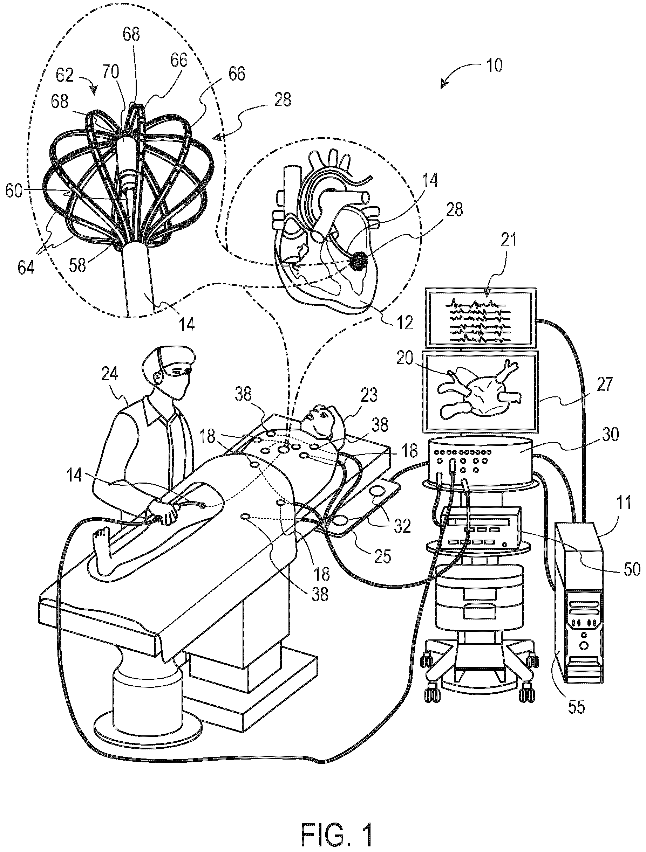

Reference is made to Figure 1, which is a simplified illustration of a catheter-based electrophysiology mapping and ablation system, according to examples of the presently disclosed subject matter, showing an example catheter-based electrophysiology mapping and ablation system 10. System 10 includes multiple catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 may place a distal tip 28 of catheter 14 in contact with the heart wall for sensing a target site in heart 12. For ablation, physician 24 may similarly place a distal end of an ablation catheter in contact with a target site for ablating tissue.

Catheter 14 is an exemplary catheter that includes one and preferably multiple electrodes 66 optionally distributed over a plurality of splines 62 at distal tip 28 and configured to sense the IEGM signals. Catheter 14 may additionally include a position sensor (not shown) embedded in or near the distal tip 28 for tracking position and orientation of distal tip 28. Optionally and preferably, the position sensor is a magnetic based position sensor including three magnetic coils for sensing three-dimensional (3D) position and orientation.

The magnetic based position sensor may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by the magnetic based position sensor. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 may include one or more electrode patches 38 positioned for skin contact on patient 23 to establish location reference for location pad 25 as well as impedance-based tracking of electrodes 66. For impedance-based tracking, electrical current is directed to electrodes 66 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 may record and display electrograms 21 captured with body surface ECG electrodes 18 and intracardiac electrograms (IEGM) captured with electrodes 66 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software stored therein, and user interface capability. Workstation 55 may provide multiple functions, optionally including:
(1) modeling the endocardial anatomy in three-dimensions (3D) and rendering a 3D graphical representation of the model or anatomical map 20 for display on a display device 27;
(2) displaying on display device 27 activation sequences (or other data) compiled from recorded electrograms 21 in representative visual indicia or imagery superimposed on the rendered anatomical map 20;
(3) modeling the catheter inserted into the body in three-dimensions (3D) and rendering a 3D graphical representation of the model for display on a display device 27;
(4) displaying real-time location and orientation of multiple catheters within the heart chamber; and
(5) displaying on display device 27 sites of interest such as places where ablation energy has been applied.

One commercial product embodying elements of the system 10 is available as the CARTO^{™} 3 System, available from Biosense Webster, Inc., 31A Technology Drive, Irvine, CA 92618.

### Various methods of measuring impedance

It is noted that the technique for impedance-based tracking of catheter location in relation to a tissue can work for various methods of measuring impedance between electrodes.

In some examples measuring impedance can be performed between one or more reference electrode(s) on a catheter, and several electrodes also in the body cavity of the patient.

In some examples the reference electrode(s) are located in or on the end assembly in a manner which precludes the reference electrode(s) touching the tissue.

In some examples measuring impedance can be performed between one or more reference electrodes transmitting an AC signal, by way of a non-limiting example an AC signal at a frequency in a kilohertz range, such as 6.4 kHz, on an end assembly of a catheter which is within a patient body cavity, and several receiving electrodes also in the body cavity of the patient.

In some examples measuring impedance can be performed between one or more electrodes on patches on a patient's body and several electrodes in a body cavity of a patient.

In some examples measuring impedance can be performed between one or more electrodes transmitting an AC electric signal on an expandable end assembly of a catheter which is within a patient body cavity, and several receiving electrodes on an external surface or splines of the expandable end assembly. In some examples, the transmitting electrode(s) are positioned on the catheter in a manner that avoids contact with the cavity wall.

In some examples measuring impedance can be performed between one or more neighboring electrodes on the distal end assembly.

### Calculation of change in impedance over time

It is noted that the technique for impedance-based tracking of catheter location in relation to a tissue can be done for a heart, while the heart is beating. When an electrode measures impedance values, to calculate a change in impedance over time, the heart wall is moving.

In some cases, the impedance is measured at a same point in time relative to the cardiac beating cycle, so that movement of the distal end assembly can be measured relative to a heart wall in a same position.

In some cases, the impedance is measured at different points in time relative to the cardiac beating cycle, and a resulting change in impedance due to catheter end assembly movement is calculated. For example, a change in maximal value of the impedance over a heartbeat cycle can indicate movement toward or away from the heart wall. Another example, a change in minimal value of the impedance over a heartbeat cycle can indicate movement toward or away from the heart wall. Another example, a change in average value of the impedance over a heartbeat cycle can indicate movement toward or away from the heart wall.

### Calculation of a direction relative to tissue

It is noted that tissue impedance is typically larger than that of blood, and a larger impedance value is expected to be measured at an electrode which is closer to tissue than at an electrode which is immersed in blood away from a tissue wall.

In some examples a normalized tissue proximity indication, normalized based on a patient-specific minimum and maximum impedance values of a patient.

In some examples a gradient is calculated based on several impedance values.

A direction of increasing values determined by the gradient is considered to be a direction toward a tissue wall. A direction of decreasing values determined by the gradient is considered to be a direction away from a tissue wall. A direction perpendicular to a direction of the gradient is considered to be substantially parallel to the tissue wall.

### Displaying

In some examples, after a direction relative to tissue, that is toward tissue, away from tissue, parallel to a tissue wall, is calculated, the direction is optionally displayed by a display.

In some examples the direction may optionally be displayed along with a virtual representation of the distal end assembly indicating its real time location and orientation in a rendered display of a 3D working volume.

In some examples a coloring of the distal device, or the electrodes on the distal device, may be used to indicate whether movement is toward a tissue wall, away from the tissue wall, or no change in impedance (potentially equivalent to movement parallel to tissue wall). Optionally, the intensity, shading or hue of the color displayed may be altered based on the magnitude and direction of the gradient.

In some examples additional graphical features may also indicate how close each electrode is to tissue. By way of a non-limiting example an electrode closest to tissue may be framed by one color and an electrode most distant from tissue may be framed by another color.

In some examples an intensity of a coloring of the distal device, or an intensity of the coloring of the electrodes on the distal device, may be used to indicate the relative proximity.

In some examples an arrow is displayed corresponding to the direction of the tissue wall.

Reference is now made to Figure 2, which is a more detailed isometric view of an expandable end assembly on the distal tip of the catheter of Figure 1.

The expandable end assembly 28 includes a coupler 26 connected to a shaft of distal end of the catheter 14, and a pusher 58 including a distal portion 60. The pusher 58 is configured to be advanced and retracted through the catheter 14, for example, using a manipulator or handle (not shown). The expandable end assembly 28 may include a plurality of splines 64. Each spline 64 may include multiple electrodes 66 disposed thereon (only some labeled for the sake of simplicity). Optionally, the expandable end assembly 28 may include a nose connector 70 connected to the distal portion 60 of the pusher 58.

In some examples, a reference electrode 59 may be attached to the pusher 59, as shown in Figure 2, or adjacent to the coupler 26, or adjacent to the nose connector 70. In some examples, more than one reference electrode 59 may be used, for example one adjacent to the coupler 26 and one adjacent to the nose connector 70. The reference electrode(s) 59 are optionally used to provide an AC signal for measuring impedance between the electrodes 66 and the reference electrode(s) 59.

Figure 2 is intended to show the electrodes 66 on splines 64 disposed in a three-dimensional (3D) dispersion in space.

In some examples, the electrodes 66 disposed along the splines 64 may be disposed in a two-dimensional (2D) dispersion in space, that is, in a plane.

Figure 2 shows some of the expandable end assembly 28 in physical contact with cavity wall tissue 80.

In some examples, the catheter and/or the expandable end assembly 28 may include one or more positions sensors embedded in a distal end of shaft 26 (not shown), for example, a position sensor (e.g., a multi-axis sensor); a position sensor disposed in the distal receptacle of the nose connector 70; and/or a position sensor (e.g., a single-axis sensor) disposed on one or more splines 54.

Before explaining at least one example of the disclosure in detail, it is to be understood that the disclosure is not necessarily limited in its application to the details of construction and the arrangement of the components and/or methods set forth in the following description and/or illustrated in the drawings and/or the Examples. The disclosure is capable of other examples or of being practiced or carried out in various ways.

Reference is now made to Figure 3, which is a simplified qualitative illustration showing impedance measured by an electrode in a body cavity as a function of distance from the cavity wall tissue according to an aspect of the disclosure.

Figure 3 shows a graph having an X-axis 302 of qualitative proximity values to cavity wall tissue and a Y-axis 304 of qualitative impedance values. The graph of Figure 3 includes a line 306 which shows qualitative values of impedance values for an electrode in a body cavity as a function of proximity to the cavity wall, and also a continuation of the line 306 into a region where the electrode touches the cavity wall at various amounts of pressure.

The graph of Figure 3 shows an expected impedance behavior in a body cavity as a function of proximity. It is noted that proximity to a cavity wall increases as distance to the cavity wall decreases.

The graph of Figure 3 shows an expected shape of the impedance proximity relationship.

In some embodiment, in-vivo measurements may determine impedance values at end points, minimum and maximum. Such minimum and maximum values may be determined per patient, optionally by accumulating data in real-time, and determining the minimum and maximum of the real-time data.

It is noted that the minimum and maximum impedance values for each electrode are, *inter alia*, a function of the electrode's distance to a reference electrode.

In some examples, the minimum and maximum impedance values per electrode are adjusted to determine normalized minimum and maximum impedance values for all the electrodes.

In some examples, data points for constructing the line 306 are optionally measured in vivo, by an electrode moving within the body cavity, measuring impedance. All or at least some of the measurements are used to produce the line 306.

In some examples, just maximum and minimum impedance values are optionally discovered, optionally per patient, by in vivo measurement.

In some examples, data points for constructing the line 306 are optionally measured in vivo, by several electrodes moving within the body cavity, the electrodes measuring impedance, and optionally a position measuring system measuring locations of the electrodes.

In some examples, data points for constructing the line 306 are optionally obtained from a database which includes data measured at some historic time for a specific patient.

In some examples, data points for constructing the line 306 are optionally obtained from a database which includes data measured at some historic time for a group of patients, optionally averaged.

Figure 3 also shows two specific levels of impedance along the Y-axis 304.

A first specific impedance level 308 shows an impedance of an electrode beginning to touch the cavity wall, and a distance to the cavity wall is close to zero. The line 306 to left of the contact impedance is in a "No Contact" impedance zone 312. In some examples, a value for the first specific impedance level 308 is determined as a specific percentage below the maximum of the line 306.

A second specific impedance level 310 shows an impedance of an electrode touching and pushing the cavity wall at a level of force which is considered sufficient for performing ablation using the electrode, if so desired. A corresponding "Contact" zone 314 is shown where the cavity wall is pushed somewhat from the point of contact due to more substantial force being applied to the cavity wall to produce sufficiently good contact. Beyond the "Contact" zone 318 the impedance flattens out, reaching its maximum, and this is shown by a "Saturation" zone 316, and a high impedance above a level referenced by 310.

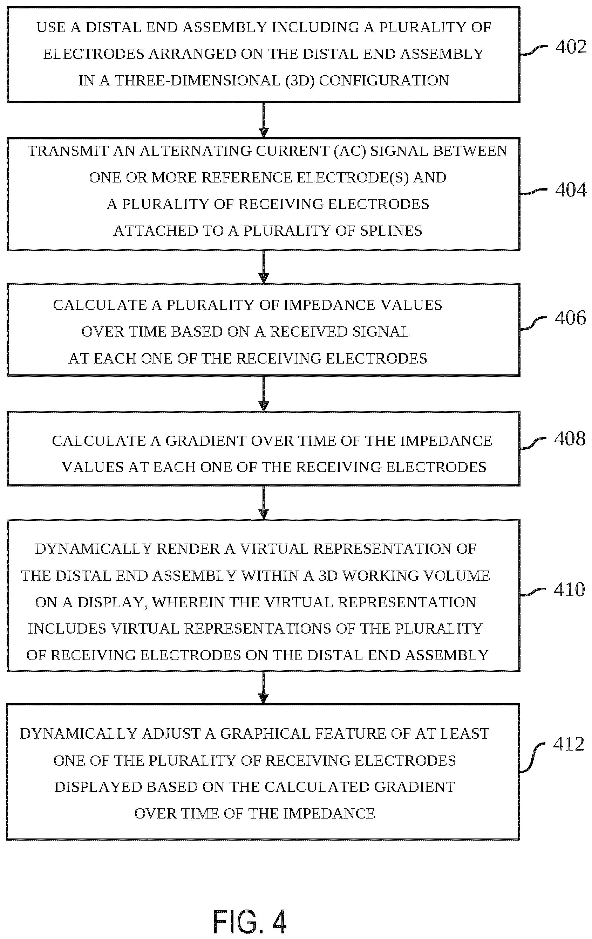

Reference is now made to Figure 4, which is a simplified flow chart illustration of a method of indicating movement of a receiving electrode of a distal end assembly with respect to a tissue cavity wall according to an aspect of the disclosure.

The method of Figure 4 includes:
using a distal end assembly comprising a plurality of electrodes arranged on the distal end assembly in a three-dimensional (3D) configuration (402), in some examples a location of the distal end assembly is tracked in a 3D working volume. The tracking may include tracking position sensor(s) at one or more of: a coupler, a pusher, a nose connector, a reference electrode, a spline, and a receiving electrode of the distal end assembly. The tracking potentially enables to render a graphical representation of the distal end assembly on a display in real-time based on the tracking. The rendering may optionally include displaying the electrodes mounted on the distal end assembly;
transmitting an alternating current (AC) signal between one or more reference electrode(s) and a plurality of receiving electrodes attached to a plurality of splines (404);
calculating a plurality of impedance values over time based on a received signal at each one of the receiving electrodes (406). In some examples the impedance values may be normalized based on distance of different receiving electrodes from the reference electrode(s) being different;
calculating a gradient over time of the impedance values at each one of the receiving electrodes (408);
dynamically rendering a virtual representation of the distal end assembly within a three-dimensional working volume on a display, wherein the virtual representation includes virtual representations of each of the plurality of receiving electrodes on the distal end assembly (410); and
dynamically adjusting a graphical feature of at least one of the plurality of receiving electrodes displayed based on the calculated gradient over time of the impedance (412).

In some examples, the dynamically adjusting includes rendering the receiving electrodes differently based on different values of the gradient over time of the impedance measured at the receiving electrodes.

The different renderings, or graphical features, corresponding to different values of the gradient can be implemented by different graphical features such as color coding, and/or shading, and/or flashing, and/or highlighting the display of the electrode(s).

In some cases, just two different types of graphical features are used to render the receiving electrodes - a first graphical feature when the value of the gradient over time is greater than a first, positive threshold, and a different, second graphical feature when the value of the impedance gradient over time is less than a second, negative threshold.

The first, positive threshold can serve to differentiate between electrodes which have greater impedance gradient values, which indicate that they are closer to the tissue cavity wall and moving toward the tissue cavity wall, and display the closer electrodes differently than the further electrodes which have a smaller value of the impedance gradient.

The second, negative threshold can serve to differentiate between electrodes which have negative impedance gradient values, which indicate that they are moving away from tissue cavity wall.

In some cases, just three different types of graphical features are used to mark the receiving electrodes - a first graphical feature when the value of the gradient over time is greater than the first, positive threshold, a different second graphical feature when the value of the gradient over time is less than a second, negative threshold, and a third graphical feature when the value of the gradient over time is between the negative threshold and the positive threshold.

In some examples, the calculating the plurality of impedance values may include normalizing the impedance values and the calculating the spatial gradient comprises using the normalized impedance values.

In some examples the normalizing may include normalizing using a previously measured maximum value and a previously measured minimum value of impedance at each one of the receiving electrodes.

The method of Figure 4 provides a visual indication to guide a physician in how to maneuver a catheter along a tissue wall of a heart chamber to map the heart chamber and/or to indicate how to maneuver the catheter to reach a location of interest.

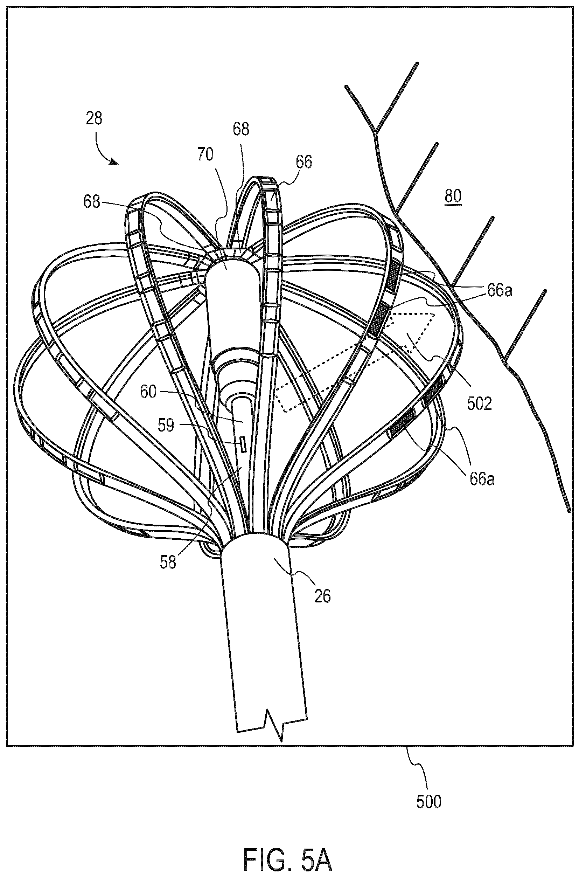

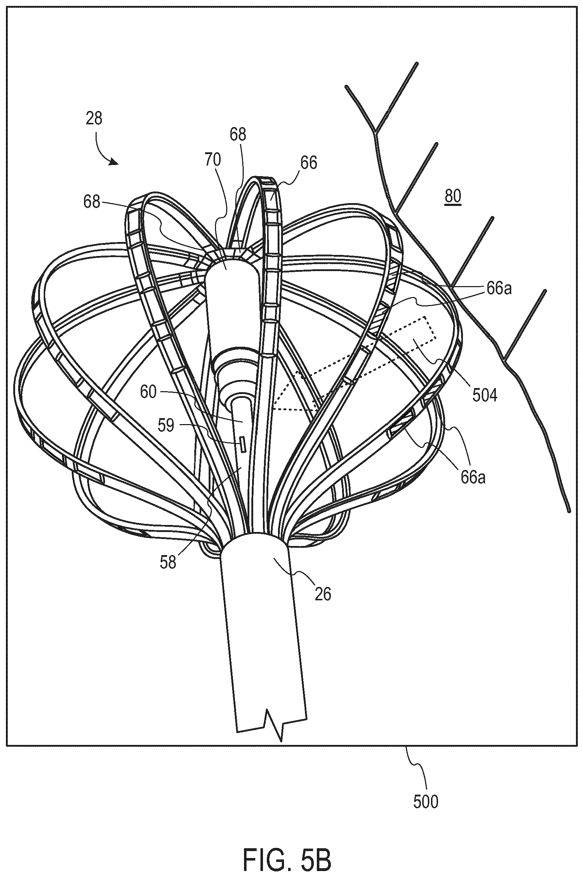

Reference is now made to Figures 5A and 5B, which are views of the end assembly of the catheter of Figure 2 as displayed on a display, and displaying a direction based on a spatial gradient superposed on the display of the expandable catheter according to an aspect of the disclosure.

Figures 5A-5C show all the components of the expandable basket-shaped distal end assembly 28 of Figure 2 shown in Figure 2, as displayed on a display 500, and where these components are shown with reference numbers, they are referenced by the same reference numbers as in Figure 2.

Figure 5A shows an arrow 502 in a direction of movement of the distal end assembly 28. The arrow 502 points toward the tissue wall 80.

The electrodes referenced as 66a are moving toward tissue wall 80, and are measuring increase of impedance, e.g. a positive gradient over time, which is greater than some positive threshold. The electrodes 66a are facing the tissue wall and moving toward the tissue, and marked by a graphical feature, for example by a first specific color indicating increase of impedance, or by a first specific brightness, or by some other indicative marking.

In some examples, the physician may be maneuvering the catheter within a cavity prior to or at the start of anatomical mapping and therefore may not be aware of the delineation of the tissue wall. The displayed indication provided on electrodes 66a may assist the physician in maneuvering the distal end assembly 28 toward a region of interest and/or along the tissue wall.

Figure 5B shows an arrow 504 in a direction of movement of the distal end assembly 28. The arrow 504 points away from the cavity wall tissue 80.

The electrodes referenced as 66a are moving away from the cavity wall tissue 80, and are measuring a decrease of impedance, e.g. a negative gradient over time, which is more negative, or lesser than, some negative threshold. The electrodes are displayed as being in the direction away from tissue and moving away from the tissue, for example by some specific color indicating the decrease of impedance, or by a specific brightness, or by some other indicative marking.

In some examples, the processor unit or processor of the workstation 55 may typically comprise a general-purpose computer, which is programmed in software, or computer program, to carry out the functions described herein. The software or computer program may be downloaded to the computer in electronic form, over a network, for example, or it may, alternatively or additionally, be provided and/or stored on non-transitory tangible media or computer-readable, storage, medium, such as magnetic, optical, or electronic memory.

### SUMMARY OF THE PRESENT DISCLOSURE

Those skilled in the art to which the present disclosure pertains, can appreciate that while the present disclosure has been described in terms of preferred examples, the concept upon which this disclosure is based may readily be utilized as a basis for the designing of other structures, systems and processes for carrying out the several purposes of the present disclosure.

Also, it is to be understood that the phraseology and terminology employed herein are for the purpose of description and should not be regarded as limiting. It should be noted that the words "comprising", "including" and "having" as used throughout the appended claims are to be interpreted to mean "including but not limited to". The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases, and disjunctively present in other cases. The term "each" may not be exclusively understood as referring to each and every, and when technically relevant may also refer to "at least some".

It is important, therefore, that the scope of the present disclosure is not construed as being limited by the illustrative examples set forth herein. Other variations are possible within the scope of the present disclosure as defined in the appended claims.

## Claims

1. A system comprising:
a display device (27); and
a processor configured to:
calculate a plurality of impedance values over time based on a received signal at each one of a plurality of receiving electrodes (66) arranged on a distal end assembly (28) in a three-dimensional configuration;
calculate a gradient over time of the impedance values at each one of the receiving electrodes (66);
dynamically render a virtual representation of the distal end assembly (28) within a three-dimensional working volume on the display device (27), wherein the virtual representation includes virtual representations of each of the plurality of receiving electrodes (66) on the distal end assembly (28); and
dynamically adjust a graphical feature of at least one of the plurality of receiving electrodes (66) displayed based on the calculated gradient over time of the impedance.

2. The system according to claim 1 wherein a first graphical feature is defined for a computed positive gradient above a defined positive threshold and a second graphical feature is defined for a computed negative gradient below a defined negative threshold.

3. The system according to claim 1 or claim 2 wherein a third graphical feature is defined for a computed gradient between the negative threshold and the positive threshold.

4. The system according to any one of claims 1-3 wherein the calculating the plurality of impedance values at each one of the receiving electrodes (66) comprises normalizing the impedance values at each one of the receiving electrodes and the calculating the impedance gradient over time comprises using the normalized impedance values.

5. The system of claim 4, wherein the normalizing comprises normalizing using a previously measured maximum value and a previously measured minimum value of impedance at each one of the receiving electrodes.

6. The system according to any one of claims 1-5, wherein the processor is configured to transmit an alternating current signal between one or more reference electrodes and the plurality of receiving electrodes attached to a plurality of splines.

## Patentansprüche

1. System, umfassend:
eine Anzeigevorrichtung (27); und
einen Prozessor, der konfiguriert ist zum:
Berechnen einer Vielzahl von Impedanzwerten im Laufe der Zeit basierend auf einem empfangenen Signal an jeder einer Vielzahl von Empfangselektroden (66), die an einer distalen Endanordnung (28) in einer dreidimensionalen Konfiguration angeordnet sind;
Berechnen eines Gradienten der Impedanzwerte im Laufe der Zeit an jeder der Empfangselektroden (66);
dynamisches Rendern einer virtuellen Darstellung der distalen Endanordnung (28) innerhalb eines dreidimensionalen Arbeitsvolumens auf der Anzeigevorrichtung (27), wobei die virtuelle Darstellung virtuelle Darstellungen jeder der Vielzahl von Empfangselektroden (66) an der distalen Endanordnung (28) einschließt; und
dynamisches Anpassen eines grafischen Merkmals von mindestens einer der Vielzahl von angezeigten Empfangselektroden (66) basierend auf dem berechneten Gradienten der Impedanz im Laufe der Zeit.

2. System nach Anspruch 1, wobei ein erstes grafisches Merkmal für einen berechneten positiven Gradienten über einer definierten positiven Schwelle definiert ist und ein zweites grafisches Merkmal für einen berechneten negativen Gradienten unter einer definierten negativen Schwelle definiert ist.

3. System nach Anspruch 1 oder 2, wobei ein drittes grafisches Merkmal für einen berechneten Gradienten zwischen der negativen Schwelle und der positiven Schwelle definiert ist.

4. System nach einem der Ansprüche 1 bis 3, wobei das Berechnen der Vielzahl von Impedanzwerten an jeder der Empfangselektroden (66) ein Normalisieren der Impedanzwerte an jeder der Empfangselektroden umfasst und das Berechnen des Impedanzgradienten im Laufe der Zeit ein Verwenden der normalisierten Impedanzwerte umfasst.

5. System nach Anspruch 4, wobei das Normalisieren das Normalisieren unter Verwendung eines zuvor gemessenen maximalen Werts und eines zuvor gemessenen minimalen Werts von Impedanz an jeder der Empfangselektroden umfasst.

6. System nach einem der Ansprüche 1 bis 5, wobei der Prozessor konfiguriert ist, um ein Wechselstromsignal zwischen einer oder mehreren Referenzelektroden und der Vielzahl von Empfangselektroden, die an einer Vielzahl von Keilen angebracht sind, zu übertragen.

## Revendications

1. Système comprenant :
un dispositif d'affichage (27) ; et
un processeur configuré pour :
calculer une pluralité de valeurs d'impédance au fil du temps sur la base d'un signal reçu au niveau de chacune d'une pluralité d'électrodes réceptrices (66) agencées sur un ensemble d'extrémité distale (28) dans une configuration tridimensionnelle ;
calculer un gradient au fil du temps des valeurs d'impédance à chacune des électrodes réceptrices (66) ;
rendre dynamiquement une représentation virtuelle de l'ensemble d'extrémité distale (28) dans un volume de travail tridimensionnel sur le dispositif d'affichage (27), dans lequel la représentation virtuelle comprend des représentations virtuelles de chacune de la pluralité d'électrodes réceptrices (66) sur l'ensemble d'extrémité distale (28) ; et
ajuster dynamiquement une caractéristique graphique d'au moins une de la pluralité d'électrodes réceptrices (66) affichées sur la base du gradient calculé au fil du temps de l'impédance.

2. Système selon la revendication 1, dans lequel une première caractéristique graphique est définie pour un gradient positif calculé supérieur à un seuil positif défini et une deuxième caractéristique graphique est définie pour un gradient négatif calculé inférieur à un seuil négatif défini.

3. Système selon la revendication 1 ou la revendication 2, dans lequel une troisième caractéristique graphique est définie pour un gradient calculé entre le seuil négatif et le seuil positif.

4. Système selon l'une quelconque des revendications 1 à 3, dans lequel le calcul de la pluralité de valeurs d'impédance au niveau de chacune des électrodes réceptrices (66) comprend la normalisation des valeurs d'impédance au niveau de chacune des électrodes réceptrices et le calcul du gradient d'impédance au fil du temps comprend l'utilisation des valeurs d'impédance normalisées.

5. Système selon la revendication 4, dans lequel la normalisation comprend une normalisation utilisant une valeur maximale mesurée précédemment et une valeur minimale mesurée précédemment d'impédance au niveau de chacune des électrodes réceptrices.

6. Système selon l'une quelconque des revendications 1 à 5, dans lequel le processeur est configuré pour transmettre un signal de courant alternatif entre une ou des électrodes de référence et la pluralité d'électrodes réceptrices fixées à une pluralité de cannelures.
